(19) Europäisches Patentamt
European Patent Office
Office européen des brevets

(11) **EP 2 683 286 B1**

(12) **FASCICULE DE BREVET EUROPEEN**

(45) Date de publication et mention
de la délivrance du brevet:
**12.11.2014 Bulletin 2014/46**

(21) Numéro de dépôt: **12712323.0**

(22) Date de dépôt: **05.03.2012**

(51) Int Cl.:
*A61B 3/08* (2006.01)          *A61B 3/113* (2006.01)
*A61B 3/14* (2006.01)          *A61B 3/10* (2006.01)
*A61B 3/00* (2006.01)

(86) Numéro de dépôt international:
**PCT/FR2012/050452**

(87) Numéro de publication internationale:
**WO 2012/123658 (20.09.2012 Gazette 2012/38)**

(54) **PROCEDE DE DETERMINATION DE L' OEIL DIRECTEUR**

VERFAHREN ZUR BESTIMMUNG DES DOMINANTEN AUGES

METHOD FOR DETERMINING THE DOMINANT EYE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **11.03.2011 FR 1152004**

(43) Date de publication de la demande:
**15.01.2014 Bulletin 2014/03**

(73) Titulaire: **Essilor International
(Compagnie Générale d'Optique)
94220 Charenton-le-Pont (FR)**

(72) Inventeur: **HADDADI, Ahmed
94220 Charenton-le-pont (FR)**

(74) Mandataire: **Allain, Laurent et al
Ipsilon Feray Lenne Conseil
63, avenue du Général Leclerc
92340 Bourg-la-Reine (FR)**

(56) Documents cités:
**WO-A1-2007/085682      WO-A1-2010/118292
US-A- 5 016 282          US-A- 5 481 622
US-A- 5 689 619**

**Description**

L'invention se rapporte à un procédé de détermination de l'oeil directeur d'un individu. L'oeil directeur d'un individu est l'oeil qui dirige l'alignement des 2 yeux, autrement dit, c'est l'oeil qui dirige le couple oculaire lors de certaines opérations, telles que, par exemple, la visée. Il est donc important, notamment pour un opticien, de connaitre l'oeil directeur d'un individu amenée à porter des lunettes.

Les procédés de détermination de l'oeil directeur d'un individu existent déjà. Habituellement, ces procédés obligent les individus à se soumettre à des tests visuels, contraignants et longs, consistant à regarder un objet à travers une ouverture pratiquée dans une feuille, et/ou à se livrer à des opérations de visée, en occultant un oeil, puis l'autre. En finalité, c'est l'individu lui-même, qui à partir de ses propres observations lors de ces tests, va déduire quel est son oeil directeur. Un opticien peut également déterminer cet oeil directeur, en observant les yeux de l'individu en cours de test, ou en analysant l'orientation de certains objets que l'individu pourrait utiliser lors desdits tests.

Les procédés de détermination de l'oeil directeur d'un individu selon l'invention, limitent les phases de contrainte pour ledit individu, en lui permettant notamment de rester les deux yeux ouverts durant le test. De plus, ils mettent en oeuvre une étape consistant à fournir à ladite personne le résultat du test, sous une forme attractive et de façon indiscutable, sans requérir la moindre interprétation. Enfin, la lecture du résultat se fait de manière quasiment instantanée, dans la continuité du test, sans période d'attente. Les procédés selon l'invention présentent donc un certain confort, et possède également un caractère démonstratif.

Le document US 5 689 619 décrit un procédé de determination de l'oeil directeur d'un individu comportant une étape de positionnement de l'individu devant un appareillage conçu pour acquérir au moins une image de face dudit individu.

L'invention se rapporte à un procédé de détermination de l'oeil directeur d'un individu, comportant les étapes suivantes :

- Positionnement de l'individu devant un appareillage conçu pour acquérir au moins une image de face dudit individu, puis pour traiter ladite image et enfin restituer une information permettant à l'individu de connaître son oeil directeur,

- Visualisation au moyen d'un dispositif de visée doté d'une fenêtre optique, d'une cible identifiable et repérable par rapport à l'appareillage, cette visualisation s'effectuant à travers ladite fenêtre et les deux yeux ouverts, les dimensions de ladite fenêtre étant telles qu'elle ne permet pas à l'individu de voir ladite cible avec les deux yeux en même temps,

- Acquisition par l'appareillage d'au moins une image permettant de visualiser au moins la position des deux yeux de l'individu,

- Traitement par calculs de ladite au moins une image, en tenant compte de la position d'un point central situé entre les deux yeux, de la position de la cible et de la position de la fenêtre,

- Restitution par l'appareillage de l'information indiquant l'oeil directeur de l'individu.

Le principe d'un procédé selon l'invention consiste à capturer au moins une image de l'individu en train de regarder la cible à travers le dispositif de visée, à traiter cette image, puis à restituer directement à l'individu l'information relative à son oeil directeur. De cette manière, le procédé est rapide, complet et démonstratif, en fournissant à l'individu une information indiscutable, dans la continuité du test. Ce procédé revêt de plus un caractère convivial, car il permet à l'individu de se mettre en scène, en gardant les yeux ouverts durant la totalité du procédé. L'information fournie par l'appareillage peut parvenir à l'individu sous différentes formes. En effet, elle peut, soit être visuelle à partir d'une image sur un écran ou sur papier révélant l'oeil directeur au moyen d'un signe distinctif, soit être sonore au moyen d'une voix préenregistrée annonçant clairement l'oeil directeur de l'individu. L'appareillage peut indifféremment mettre en oeuvre un appareil photo ou une caméra. Cet appareillage peut, soit être mobile et être déplacé durant le procédé pour acquérir au moins une image, soit être fixe sous la forme, par exemple, d'une colonne incluant également les moyens de traitement de l'image et les moyens de restitution de ladite image. L'individu peut exécuter le test en étant debout ou bien en étant assis. Dans le cas où l'individu est assis, le siège peut être fixe, figeant de façon précise et répétitive la position de l'individu par rapport à l'appareillage. La cible est fixe et son lieu d'implantation est parfaitement repérable. Elle est généralement située dans l'environnement immédiat de l'appareillage, de sorte qu'en se plaçant devant ledit appareillage, l'individu puisse l'apercevoir directement, sans tourner la tête. Le dispositif de visée peut, par exemple, être constitué par une pièce inamovible, posée sur le sol, à un emplacement étudié entre l'individu et l'appareillage. La fenêtre optique est un élément local sur le dispositif de visée, au travers duquel la personne peut regarder la cible, ladite fenêtre étant dimensionnée pour que la personne ne puisse voir la cible que d'un seul oeil. Les images fournies par l'appareillage doivent être suffisamment précise, pour que les moyens de traitement puissent repérer un point central situé entre les deux yeux de l'individu, ainsi que la position de la fenêtre optique.

Préférentiellement, le dispositif de visée est portable, l'individu ajustant manuellement la position de ce dispositif pour voir la cible. Le dispositif de visée est suffisamment léger et petit, pour que l'individu puisse le manipuler aisément avec la main, sans avoir à fournir d'efforts particuliers. Pour cette configuration, l'individu positionne le dispositif de visée à sa convenance, dans une position pour laquelle il se sent à l'aise pour voir la cible. Généralement, l'individu place le dispositif de visée à quelques dizaines de centimètres de ses yeux, préférentiellement entre 30cm et 60cm, comme il le ferait pour lire un livre.

Avantageusement, le moyen d'acquisition de l'image est une caméra. Ce moyen est privilégié, car il est souple d'utilisation, et qu'il autorise une multiplicité de prises de vue en continu, de façon éloignée ou rapprochée. Ce moyen fournit de plus des images particulièrement adaptées au traitement proposé par le procédé.

Selon un premier mode de réalisation préféré d'un procédé selon l'invention, les deux yeux de l'individu ainsi que la fenêtre optique apparaissent simultanément sur la même image. Pour cette configuration, il est supposé que le moyen d'acquisition de l'image possède une assez bonne résolution, pour pouvoir repérer précisément les différents éléments nécessaires au traitement de ladite image par calcul, comme la fenêtre optique du dispositif de visée, et le point situé entre les deux yeux de l'individu.

Selon un deuxième mode de réalisation préféré d'un procédé selon l'invention, l'appareillage acquiert au moins deux images, une première image qui est une vue éloignée sur laquelle figurent à la fois les yeux de l'individu et la fenêtre optique, et une deuxième image qui est une vue zoomée des yeux de l'individu et qui permet d'identifier précisément la position d'un point central situé entre les yeux, le traitement de la première image s'effectuant à partir des informations recueillies sur la deuxième image. Souvent, il arrive que la résolution des moyens d'acquisition de l'image ne soit pas suffisante pour pouvoir repérer sur une même image, la position des différents éléments nécessaires au traitement par calcul. Dans ce cas, il peut être effectuée une vue zoomée des yeux de l'individu permettant de repérer précisément un point central situé entre lesdits yeux. Une fois ce repérage effectué, cette vue zoomée est combinée à la première image sur laquelle apparaissent à la fois les yeux de l'individu ainsi que la position de la fenêtre optique du dispositif de visée.

De façon préférentielle, l'individu porte une paire de lunettes, sur laquelle est fixé un clip équipé d'au moins un marqueur, dont au moins un est destiné à repérer un point central entre les yeux. En effet, pour aider au repérage d'un point central situé entre les yeux de l'individu, il peut être utilisé un marqueur artificiel, permettant de repérer plus facilement ledit point central. Généralement, ce marqueur est identifiable par sa forme et/ou sa couleur. D'autres marqueurs latéraux peuvent être utilisés, pour notamment repérer, de façon précise, la position de chaque oeil. Le fait d'avoir recours à un clip, permet d'avoir un repère lié à la tête. Un tel clip est, par exemple, décrit dans la demande de brevet US2009/0262302.

De façon avantageuse, le traitement par calcul se fonde sur la position d'une première droite joignant la cible et la fenêtre optique, et d'une deuxième droite reliant ladite cible à un point central situé entre les deux yeux de l'individu, la détermination desdites positions s'effectuant par rapport à une droite de référence, passant par la cible et étant parallèle à l'axe de visée du moyen d'acquisition de l'image. Le positionnement de ces droites sera d'autant plus précis, que le repérage de la fenêtre optique et du point central entre les deux yeux sera fin. Si la cible et le moyen d'acquisition de l'image sont alignés selon un axe vertical, alors la droite de référence et l'axe de visée dudit moyen d'acquisition sont confondus.

De façon plus précise, le traitement par calcul suit au moins deux étapes, dont la première consiste à déterminer un premier angle entre la première droite et la droite de référence, et un deuxième angle entre la deuxième droite et ladite droite de référence, et dont la deuxième étape consiste à comparer lesdits angles entre eux.

Préférentiellement, l'appareillage met en oeuvre un écran de visualisation délivrant une image traitée sur laquelle apparaît en incrustation, au moins un identifiant indiquant l'oeil directeur de l'individu. Cette manière de restituer l'information permet à l'individu de se voir sur une image, agrémentée de l'information permettant d'identifier son oeil directeur. L'identifiant est une trace ou une marque pouvant revêtir une multiplicité de formes, de tailles ou de couleurs indiquant sans ambiguïté l'oeil directeur de l'individu.

Avantageusement, l'identifiant est un trait joignant l'oeil directeur de l'individu à la fenêtre optique du dispositif de visée. En mode vidéo les images peuvent, par exemple, être sélectionnées et/ou être restituées sous la forme d'un film traité au moyen de la réalité augmentée, sur lequel apparait le visage de l'individu en mouvement avec le trait se déplaçant en même temps que l'oeil directeur.

De façon préférentielle, le dispositif de visée est doté d'au moins un marqueur de repérage, dont l'un au moins permet d'identifier la position de la fenêtre optique. De cette manière, quelle que soit la position du dispositif de visée, au moment où l'individu visualise la cible à travers la fenêtre optique, la position de la fenêtre optique sera toujours visible sur l'image. D'autres marqueurs latéraux peuvent également être apposés sur ledit dispositif, pour repérer son orientation sur l'image.

De façon avantageuse, la fenêtre optique est un orifice traversant. Il s'agit de la version la plus simple, la plus rapide à mettre en oeuvre et la moins coûteuse. Selon d'autres modes de réalisation préférés, cette fenêtre optique peut être matérialisée par une épaisseur de verre ou de plastique transparent, ledit verre ou ledit plastique pouvant être coloré. De même, cette fenêtre optique peut être occultée entièrement ou partiellement par un organe d'occultation coulissant, permettant d'adapter les dimensions de cette fenêtre aux contraintes rencontrées.

De façon préférentielle, l'appareillage est une colonne fixe, la cible étant placée sur ladite colonne. De cette manière, les éléments nécessaires à mettre en oeuvre le procédé selon l'invention, sont tous regroupés et le procédé peut alors se dérouler dans un espace d'encombrement réduit.

L'invention se rapporte également à un dispositif de visée portable pour la mise en oeuvre d'un procédé selon l'invention. La principale caractéristique d'un dispositif de visée selon l'invention est qu'il comprend une tablette de faible épaisseur comportant une fenêtre optique, les dimensions de ladite tablette étant compatibles avec une manipulation manuelle aisée, la tablette comprenant un socle possédant au moins un marqueur représentatif de la position de la fenêtre optique pour permettre de repérer ladite fenêtre sur une image dans le cas où la tablette serait inclinée.

Avantageusement, la fenêtre optique est dimensionnée pour qu'un individu ne puisse pas voir la cible avec les deux yeux en même temps, la tablette recouvrant partiellement le cône optique, dont la base est délimitée par les deux yeux de l'individu, et dont l'extrémité correspond à la cible.

Les procédés de détermination de l'oeil directeur d'une personne selon l'invention, présentent l'avantage d'être complets, en proposant, outre la mise en oeuvre d'un test simple, rapide et peu contraignant, une ultime étape consistant à restituer à la personne, le résultat du test sous une forme visuelle et précise. De plus les procédés selon l'invention, sont avantageux à deux titres, d'une part, car l'individu se met en scène devant une caméra, en plaçant le cas échéant un clip sur ses lunettes, et d'autre part, qu'il peut se voir sur un écran ou sur un cliché papier avec l'indication de son oeil directeur. Enfin, ces procédés sont sûrs, fiables et parfaitement répétitifs.

On donne ci-après, un mode de réalisation préféré d'un procédé de détermination de l'oeil directeur selon l'invention en se référant aux figures 1 à 4.

- La figure 1 est une vue latérale d'une installation prévue pour appliquer le procédé selon l'invention, et montrant un individu tentant de visualiser une cible à travers un dispositif de visée portable,

- La figure 2a est une vue en perspective de coté d'un dispositif de visée portable, utilisé pour la mise en oeuvre d'un procédé selon l'invention,

- La figure 2b est une vue en perspective de face du dispositif de la figure 2a,

- La figure 3a est une vue du dessus de l'installation de la figure 1, et montrant la personne tentant de visualiser une cible à travers une tablette, la cible étant placée au droit d'une caméra d'acquisition.

- La figure 3b est une vue du dessus de l'installation de la figure 1, et montrant la personne tentant de visualiser une cible à travers une tablette, la cible étant décalée horizontalement par rapport à une caméra d'acquisition.

- La figure 4 est une vue de l'image finale proposée par le procédé selon l'invention, et montrant une personne visualisant une cible à travers une tablette, ladite image étant agrémentée d'une trace en incrustation désignant l'oeil directeur de ladite personne.

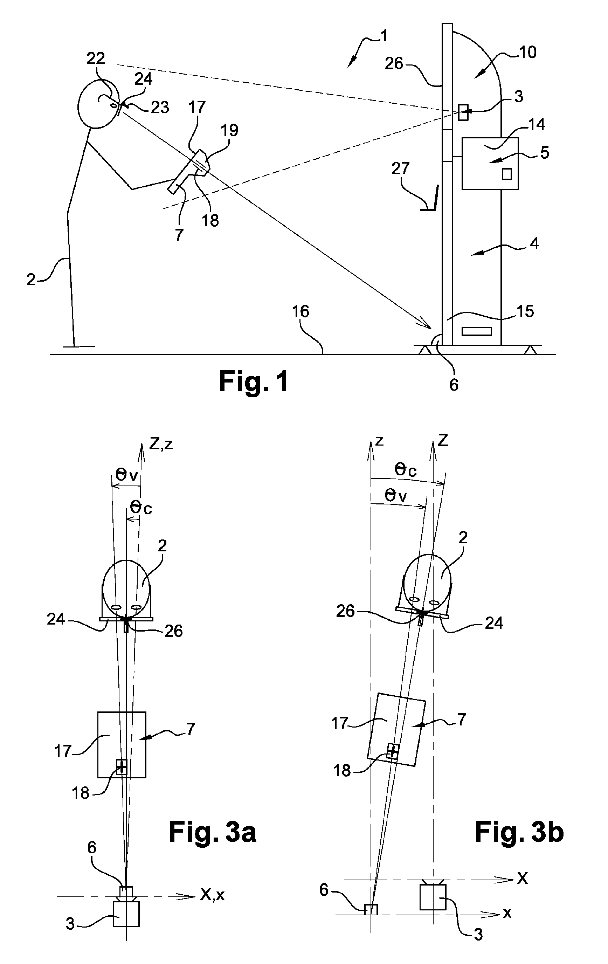

En se référant aux figures 1, 3a et 3b, une installation 1 permettant d'appliquer un procédé de détermination de l'oeil directeur d'un individu 2 selon l'invention, fait intervenir un appareillage 10 doté d'une caméra 3 permettant d'acquérir au moins une image 13, de moyens 4 permettant de traiter ladite image 13 et de moyens 5 permettant de restituer ladite image 13 traitée, d'un système d'éclairage dudit individu 2, d'une cible 6 matérielle et fixe, dont l'emplacement est parfaitement identifié et repéré, d'un dispositif de visée 7 portable tenue à la main par l'individu 2 et de différents marqueurs 20,21,23,26 permettant de repérer la position du dispositif de visée 7 et de chaque oeil de l'individu 2 sur la photo ou le film délivré par la caméra 3. L'appareillage se présente sous la forme d'une colonne 10 verticale et fixe, intégrant la caméra 3, dont la hauteur est ajustable en fonction de la taille de l'individu 2 se soumettant au test. Idéalement la hauteur de la caméra 3 est positionnée approximativement à la hauteur à laquelle se situent les yeux de l'individu 2. Les moyens de traitement 4 de l'image 13 comportent une composante électronique et une composante informatique, permettant notamment d'incruster une trace 11 colorée sur la photo 13 ou le film, acquis par la caméra 3, cette trace 11 étant élaborée à partir de la position d'un point central 26 situé entre les yeux de l'individu 2, de la position du dispositif de visée 7, et de la position de la cible 6, ladite trace 11 désignant ainsi directement l'oeil 12 directeur de l'individu 2. Les moyens 5 de restitution de l'image traitée 13, comportent un écran 14 de visualisation, qui peut, soit être directement incorporé dans la colonne 10, soit être relié à celle-ci par les cordons appropriés. Avantageusement l'écran 14 est orienté vers l'individu, afin qu'il puisse directement visualiser sa propre image 13, et découvrir, de façon quasiment instantanée, et sans bouger, quel est son oeil directeur 12. La cible 6 est constituée d'un repère visible pouvant revêtir toute sorte de forme, et est positionnée à la partie inférieure 15 de la colonne 10, le long d'un axe de symétrie vertical séparant fictivement ladite colonne 10 en deux parties égales. L'individu 2 peut se soumettre au test de détection de son oeil directeur 12 selon l'invention, en étant en position debout ou en position assise. Lorsqu'il est debout, la cible 6 est placée à une hauteur d'environ 70 cm du sol 16. Lorsqu'il est assis, la cible 6 est placée au niveau du sol 16. Préférentiellement, la caméra 3 est positionnée dans la colonne 10 de façon à être également traversé de façon symétrique par ledit axe vertical, de sorte que ladite caméra 3 et ladite cible 6 se retrouvent parfaitement au droit l'un de l'autre, en étant parfaitement alignés le long dudit axe vertical. L'individu 2 qui souhaite connaître son oeil directeur 12, se saisit du dispositif de visée 7 pour visualiser à travers celle-ci la cible 6. La colonne 10 dispose d'un miroir 26 sans tain et plan, camouflant la caméra 3 et permettant à la personne 2 de se positionner correctement devant ladite colonne 10, par référence à sa propre image renvoyée par ledit miroir 26, pour effectuer le test dans de bonnes conditions. La colonne 10 comprend également un support 27 en saillie, permettant de poser le dispositif de visée 7 lorsque celui-ci n'est pas utilisé.

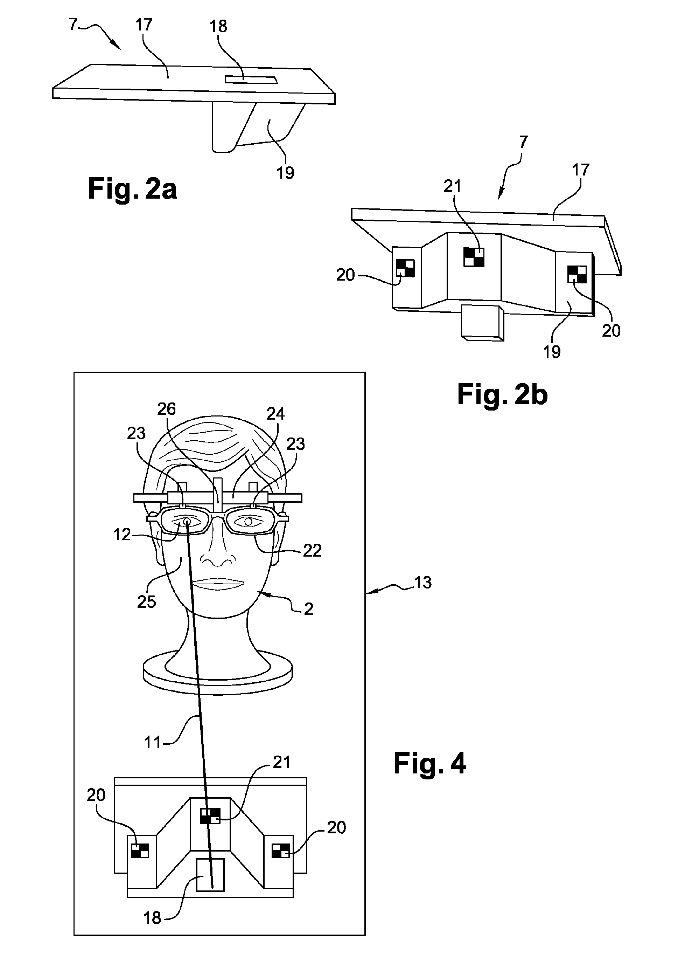

En se référant aux figures 2a et 2b, un dispositif de visée 7 portable, et pouvant être utilisée dans le cadre du procédé selon l'invention, se présente sous la forme d'une tablette 17 rectangulaire, préférentiellement de 20 cm sur 30 cm, ayant une épaisseur inférieure à 5cm, et dotée d'une fenêtre optique 18, ladite tablette 17 comportant un socle 19 sur lequel sont apposés trois marqueurs 20,21 permettant de repérer sur une photo 13 ou un film, la position de ladite tablette 7, deux marqueurs 20 étant en position latérale et l'autre marqueur 21 étant en position centrale sur le dispositif 7. Chaque marqueur 20,21 est constitué d'un carré partagé en quatre petits carrés identiques, dont deux carrés en diagonale sont de couleur blanche, les deux autres carrés étant de couleur noire. Avantageusement, pour une meilleure détection en fonction de l'éclairement ambiant, et afin d'éviter une saturation, les tons de ces marqueurs 20,21 peuvent être gris clair et gris foncé. Le marqueur central 21 est représentatif de la position de la fenêtre optique 18 sur la tablette 7. En effet, lorsque l'individu 2 va essayer d'apercevoir la cible 6 à travers le dispositif de visée 7, il va avoir tendance à incliner celui-ci, avec le risque que la fenêtre optique 18 disparaisse de la photo 13 ou du film pris par la caméra 3. Puisque le traitement par calcul de la photo 13 ou du film, repose en partie sur la position de cette fenêtre optique 18, il est important de pouvoir la repérer précisément, quelle que soit l'inclinaison dudit dispositif de visée 7. Le marqueur central 21 est positionné sur le socle 19 pour répondre à ce besoin. Les deux marqueurs 20 latéraux contribuent au repérage de l'orientation du dispositif de visée 7. La fenêtre optique 18 représente un élément situé sur le dispositif de visée 7, et au travers duquel l'individu 2 va visualiser la cible 6. Cette fenêtre optique 18 est préférentiellement matérialisée par un trou pratiqué dans la tablette 17 et traversant celle-ci, mais peut également être constituée d'une épaisseur d'un matériau transparent, de type verre ou plastique, ce matériau pouvant être coloré. De même, cette fenêtre optique 18 peut être de dimension ajustable, par l'intermédiaire, par exemple, d'un élément d'occultation coulissant intégré à la tablette 17. Pour une utilisation dans le cadre d'un procédé selon l'invention, la fenêtre optique 18 doit être dimensionnée de telle manière que la personne ne puisse pas voir la cible 6 à travers cette fenêtre 18 avec les deux yeux en même temps. L'individu se positionne à environ 1,5m de la caméra et place le dispositif de visée 7 à une distance d'observation comprise entre 30 à 60 cm, l'ouverture du trou 18 ayant une longueur ou un diamètre compris entre 20 et 30 mm. Ainsi la position de la fenêtre 18 par rapport à l'individu 2 sera telle, que seul l'oeil directeur 12 pourra observer la cible 6, même si l'image 13 de la scène est intégrée par le système visuel 3. Par conséquent cela conduit à avoir un positionnement latéral de la fenêtre optique 18 par rapport à l'oeil directeur 12.

Préférentiellement, dans le cadre d'une prise de mesure chez l'opticien, l'individu 2 porte une monture 22 de verres correcteurs. Puisque le principe du procédé repose sur l'acquisition d'une image traitée 13 à partir de l'identification précise de la position d'un point central situé entre les yeux, de la position de la fenêtre optique 18 de la tablette 17, et de la position de la cible 6, il est donc important de pouvoir repérer de façon rigoureuse, la position dudit point central et le cas échéant celle de chaque oeil. Pour ce faire, les lunettes 22 sont équipées de marqueurs 23,26, dont deux 23 sont situés au bord de la monture, et dont le troisième 26 est situé au niveau d'un point central situé entre les yeux. Ces trois marqueurs 23,26 sont supportés par une tige 24 pouvant venir se clipper sur la monture desdites lunettes 22, dans une position sensiblement horizontale, au dessus de chaque verre, ce clip pouvant, par exemple, être analogue à celui décrit dans la demande de brevet US2009/0262302. Les marqueurs 23,26 apposés sur la monture 22, peuvent être analogues à ceux 20,21 de la tablette de visée 17, mais peuvent également revêtir d'autres formes, qui seraient plus adaptées à un environnement de type paire de lunettes 22. Une fixation par clippage est favorisée pour accroitre le coté souple, rapide et attractif d'un procédé selon l'invention. Le système d'éclairage de l'individu 2 est conventionnel et est solidarisé à la colonne 10 constituant l'appareillage, ce système d'éclairage étant destiné à mettre en relief les contrastes sur une photo ou sur un film, afin de mieux repérer certains éléments du visage de l'individu 2, comme par exemple, les différents marqueurs 23,26.

En se référant aux figures 3a et 3b, le traitement par calcul de l'image 13, s'appuie sur le repérage de la position du marqueur 26 situé entre les deux yeux de l'individu 2, de la position de la fenêtre optique 18 ou du marqueur 21 permettant de l'identifier, ainsi que de la position de la cible 6. Si les positions du point central 26 et de la fenêtre optique 18 peuvent être repérées par la caméra, en revanche il n'en est pas de même pour la cible 6 qui n'apparait pas sur les images 13. Il est donc impératif de bien identifier les coordonnées de cette cible 6 pour traiter lesdites images. La figure 3a illustre une configuration parfaitement symétrique, pour laquelle la cible 6 et la caméra 3 sont strictement alignées selon un axe vertical. La figure 3b illustre une configuration dissymétrique pour laquelle la cible 6 et la caméra 3 sont décalées horizontalement. Pour les deux configurations, (X,Z) est un repère situé dans un plan horizontal et ayant pour origine le point de visée situé sur l'optique de la caméra 3. L'axe Z matérialise l'axe de visée de ladite caméra, et l'axe X un axe perpendiculaire à cet axe Z de visée.

L'angle θv est l'angle formé entre la droite parallèle à l'axe Z passant par le centre de la cible 6, et la droite passant par le centre de la cible 6 et la fenêtre optique 18 du dispositif de visée 7.

L'angle θc est l'angle formé entre la droite parallèle à l'axe Z passant par le centre de la cible 6 et la droite passant par le marqueur 26 du clip 24 matérialisant un point central situé entre les deux yeux.

Le sens des angles θv et θc est le sens trigonométrique.

Dans ces conditions :

- si θv > θc, l'oeil directeur est l'oeil droit,
- si θv < θc, l'oeil directeur est l'oeil gauche,

Le calcul de θv et θc s'effectue en utilisant les coordonnées dans le plan (x,z), x étant parallèle à X et z étant parallèle à Z, l'origine de ce repère (x,z) étant un point central de la cible 6,

- du centre de la fenêtre optique 18 (xv, zv)

- du marqueur 26 représentatif d'un point central situé entre les yeux de l'individu 2 (xc, zc),

- du centre de la cible 6 (xr, zr).

Ainsi, il est aisé de déduire les relations suivantes :

$$\theta v = \arctan((zv-zr)/(xv-xr))$$

$$\theta c = \arctan((zc-zr)/(xc-xr))$$

En se référant à la figure 4, l'image traitée 13 fournie par la colonne 10, est une photo permettant de visualiser le visage 25 de l'individu 2, portant des lunettes 22 munies de la tige 24 dotée de marqueurs 23,26, ainsi que le dispositif de visée 7 également dotés de ses marqueurs 20,21 visibles. Le traitement de l'image 13 consiste, dans un premier temps, à repérer la position du marqueur 26 représentatif d'un point central situé entre les deux yeux, ainsi que celle du marqueur 21 représentatif de la fenêtre optique 14, puis, pour démontrer à l'individu sa direction de visée, de tracer en incrustation sur la photo 13, une droite colorée 11 joignant l'oeil directeur 12 au marqueur central 21 du dispositif de visée 7, et représentatif de la position de la fenêtre optique 18.

Un procédé selon l'invention met en oeuvre, les étapes suivantes :

- Fixation du clip 24,23,26 sur les lunettes 22 de l'individu 2 afin de pouvoir repérer, sur une photo 13 ou un film, un point central 26 entre les deux yeux,

- Positionnement de l'individu 2 devant la colonne 10 d'acquisition, à une distance d'environ 1,3m, en se référant à sa propre image reflétée par le miroir 26,

- Acquisition par l'individu 2 du dispositif de visée 7 muni de sa fenêtre optique 18,

- Ajustement de la position du dispositif de visée 7, pour regarder ensuite la cible 6 à travers la fenêtre optique 18, en maintenant les deux yeux ouverts, cet ajustement étant effectué à la convenance de l'individu 2,

- Déclenchement de la caméra 3, puis arrêt sur une image 13 pour figer le visage 25 de l'individu 2 ainsi que la position du dispositif de visée 7,

- Traitement de ladite image 13,

- Restitution par l'écran 14 d'une image traitée 13, sur laquelle apparaît, en incrustation, une droite colorée 11 joignant l'oeil directeur 12 de l'individu 2, au marqueur 21 central du dispositif de visée 7 et qui est représentatif de la position de la fenêtre optique 18,

Le procédé peut également mettre en oeuvre une ultime étape consistant à effectuer un tirage papier de l'image 13 sur laquelle apparait le visage 25 de l'individu 2, ainsi que le trait coloré indiquant son oeil directeur 12.

Le procédé selon l'invention peut prévoir l'ajout séquentiel de masques occultant un oeil puis l'autre, et d'observer le déplacement latéral de la fenêtre optique 18 à l'aide de séquence d'images 13 acquises.

L'invention ne se limite aux différents modes de réalisation décrits ci-avant, et donnés à titre d'exemples non limitatifs.

**Revendications**

1. Procédé de détermination de l'oeil directeur (12) d'un individu (2), comportant les étapes suivantes :

   - Positionnement de l'individu (2) devant un appareillage (10) conçu pour acquérir au moins une image (13) de face dudit individu (2), puis pour traiter ladite image (13) et enfin restituer une information permettant à l'individu (2) de connaître son oeil directeur (12),
   - Visualisation au moyen d'un dispositif de visée (7) doté d'une fenêtre optique (18), d'une cible (6) identifiable et repérable par rapport à l'appareillage (10), cette visualisation s'effectuant à travers ladite fenêtre (18) et les deux yeux ouverts, les dimensions de ladite fenêtre (18) étant telles qu'elle ne permet pas à l'individu (2) de voir ladite cible (6) avec les deux yeux en même temps,

- Acquisition par l'appareillage (10) d'au moins une image (13) permettant de visualiser au moins la position des deux yeux de l'individu (2) et de la fenêtre optique (18),

- Traitement par calculs de ladite au moins une image (13), en tenant compte de la position d'un point central situé entre les deux yeux de l'individu (2), de la position de la cible (6) et de la position de la fenêtre (18),

- Restitution par l'appareillage (10) de l'information indiquant l'oeil directeur (12) de l'individu (2).

2. Procédé selon la revendication 1, **caractérisé en ce que** le dispositif de visée (7) est portable, et **en ce que** l'individu (2) ajuste manuellement la position de ce dispositif (7) pour voir la cible (6).

3. Procédé selon l'une quelconque des revendications 1 ou 2, **caractérisé en ce que** le moyen d'acquisition de l'image est une caméra (3).

4. Procédé selon l'une quelconque des revendications 1 à 3, **caractérisé en ce que** les deux yeux de l'individu (2) ainsi que la fenêtre optique (18) apparaissent simultanément sur la même image (13).

5. Procédé selon l'une quelconque des revendications 1 à 3, **caractérisé en ce que** l'appareillage (10) acquiert au moins deux images (13), une première image qui est une vue éloignée sur laquelle figurent à la fois les yeux de l'individu (2) et la fenêtre optique (18), et une deuxième image qui est une vue zoomée des yeux de l'individu (2) et qui permet d'identifier précisément la position d'un point central situé entre les yeux, le traitement de la première image s'effectuant à partir des informations recueillies sur la deuxième image.

6. Procédé selon l'une quelconque des revendications 1 à 5, **caractérisé en ce que** l'individu (2) porte une paire de lunettes (22), sur laquelle est fixé un clip (24) équipé d'au moins un marqueur (23,26), dont au moins un est destiné à repérer un point central entre les yeux.

7. Procédé selon l'une quelconque des revendications 1 à 6, **caractérisé en ce que** le traitement par calcul se fonde sur la position d'une première droite joignant la cible (6) et la fenêtre (18), et d'une deuxième droite reliant ladite cible (6) à un point central situé entre les deux yeux, la détermination desdites positions s'effectuant par rapport à une droite de référence passant par la cible (6) et étant parallèle à l'axe de visée du moyen d'acquisition (3) de l'image.

8. Procédé selon la revendication 7, **caractérisé en ce que** le traitement par calcul suit au moins deux étapes, dont la première consiste à déterminer un premier angle ($\theta v$) entre la première droite et la droite de référence, et un deuxième angle ($\theta c$) entre la deuxième droite et ladite droite de référence, et dont la deuxième étape consiste à comparer lesdits angles entre eux.

9. Procédé selon l'une quelconque des revendications 1 à 8, **caractérisé en ce que** l'appareillage (10) met en oeuvre un écran (14) de visualisation délivrant une image traitée (13) sur laquelle apparait en incrustation, au moins un identifiant (11) indiquant l'oeil directeur (12) de l'individu (2).

10. Procédé selon la revendication 9, **caractérisé en ce que** l'identifiant est un trait (11) joignant l'oeil directeur (12) de l'individu (2) à la fenêtre optique (18) du dispositif de visée (7).

11. Procédé selon l'une quelconque des revendications 1 à 10, **caractérisé en ce que** le dispositif de visée (7) est doté d'au moins un marqueur (20,21) de repérage, dont l'un (21) au moins permet d'identifier la position de la fenêtre optique (18).

12. Procédé selon l'une quelconque des revendications 1 à 11, **caractérisé en ce que** la fenêtre optique est un orifice traversant (18).

13. Procédé selon l'une quelconque des revendications 1 à 12, **caractérisé en ce que** l'appareillage est une colonne fixe (10), et **en ce que** la cible (6) est placée sur ladite colonne (10).

14. Dispositif de visée (7) portable pour la mise en oeuvre d'un procédé selon l'une quelconque des revendications 1 à 13, **caractérisé en ce qu'**il comprend une tablette (17) de faible épaisseur comportant une fenêtre optique (18), les dimensions de ladite tablette (17) étant compatibles avec une manipulation manuelle aisée, et **en ce que** la tablette (17) comprend un socle (19) possédant au moins un marqueur (21) représentatif de la position de la fenêtre optique (18), pour permettre de repérer ladite fenêtre (18) sur une image (13) dans le cas où la tablette (17) serait inclinée.

15. Dispositif selon la revendication 14, **caractérisé en ce que** la fenêtre optique (18) est dimensionnée pour qu'un individu (2) ne puisse pas voir la cible (6) avec les deux yeux en même temps, la tablette (17) recouvrant partiellement le cône optique, dont la base serait délimitée par les deux yeux de l'individu (2), et dont la pointe correspondrait à la cible (6).

**Patentansprüche**

1. Verfahren zur Bestimmung des dominanten Auges (12) einer Person (2), das die folgenden Schritte aufweist:

   - Positionieren der Person (2) vor einem Gerät (10), das konzipiert ist, um mindestens ein Bild (13) der Person (2) von vorne zu erfassen, um dann das Bild (13) zu verarbeiten und schließlich eine Information wiederzugeben, die es der Person (2) erlaubt, ihr dominantes Auge (12) zu kennen,
   - Sichtbarmachung, mittels einer mit einem optischen Fenster (18) versehenen Zielvorrichtung (7), eines bezüglich des Geräts (10) erkennbaren und anpeilbaren Ziels (6), wobei diese Sichtbarmachung durch das Fenster (18) und mit beiden Augen offen erfolgt, wobei die Abmessungen des Fensters (18) so sind, dass es der Person (2) nicht erlaubt, das Ziel (6) mit beiden Augen gleichzeitig zu sehen,
   - Erfassung durch das Gerät (10) mindestens eines Bilds (13), das es ermöglicht, zumindest die Position der zwei Augen der Person (2) und des optischen Fensters (18) sichtbar zu machen,
   - Verarbeitung durch Berechnungen des mindestens einen Bilds (13) unter Berücksichtigung der Position eines zwischen den zwei Augen der Person (2) befindlichen zentralen Punkts, der Position des Ziels (6) und der Position des Fensters (18),
   - Wiedergabe durch das Gerät (10) der Information, die das dominante Auge (12) der Person (2) angibt.

2. Verfahren nach Anspruch 1, **dadurch gekennzeichnet, dass** die Zielvorrichtung (7) tragbar ist, und dass die Person (2) die Position dieser Vorrichtung (7) manuell einstellt, um das Ziel (6) zu sehen.

3. Verfahren nach einem der Ansprüche 1 oder 2, **dadurch gekennzeichnet, dass** die Erfassungseinrichtung des Bilds eine Kamera (3) ist.

4. Verfahren nach einem der Ansprüche 1 bis 3, **dadurch gekennzeichnet, dass** die zwei Augen der Person (2) sowie das optische Fenster (18) gleichzeitig im gleichen Bild (13) erscheinen.

5. Verfahren nach einem der Ansprüche 1 bis 3, **dadurch gekennzeichnet, dass** das Gerät (10) mindestens zwei Bilder (13) erfasst, ein erstes Bild, das eine entfernte Ansicht ist, in der gleichzeitig die zwei Augen der Person (2) und das optische Fenster (18) zu sehen sind, und ein zweites Bild, das eine gezoomte Ansicht der Augen der Person (2) ist und es ermöglicht, die Position eines zwischen den Augen befindlichen zentralen Punkts genau zu erkennen, wobei die Verarbeitung des ersten Bilds ausgehend von den im zweiten Bild erhaltenen Informationen ausgeführt wird.

6. Verfahren nach einem der Ansprüche 1 bis 5, **dadurch gekennzeichnet, dass** die Person (2) eine Brille (22) trägt, an der ein Clip (24) befestigt ist, der mit mindestens einer Markierung (23, 26) ausgestattet ist, von denen mindestens eine dazu bestimmt ist, einen zentralen Punkt zwischen den Augen anzupeilen.

7. Verfahren nach einem der Ansprüche 1 bis 6, **dadurch gekennzeichnet, dass** die Verarbeitung durch Berechnung auf der Position einer ersten Geraden, die das Ziel (6) und das Fenster (18) verbindet, und einer zweiten Geraden beruht, die das Ziel (6) mit einem zwischen den zwei Augen befindlichen zentralen Punkt verbindet, wobei die Bestimmung der Positionen bezüglich einer Bezugsgeraden erfolgt, die durch das Ziel (6) geht und zur Zielachse der Erfassungseinrichtung (3) des Bilds parallel ist.

8. Verfahren nach Anspruch 7, **dadurch gekennzeichnet, dass** die Verarbeitung durch Berechnung mindestens zwei Schritten folgt, von denen der erste darin besteht, einen ersten Winkel ($\theta v$) zwischen der ersten Geraden und der Bezugsgeraden und einen zweiten Winkel ($\theta c$) zwischen der zweiten Geraden und der Bezugsgeraden zu bestimmen, und von denen der zweite Schritt darin besteht, die Winkel miteinander zu vergleichen.

9. Verfahren nach einem der Ansprüche 1 bis 8, **dadurch gekennzeichnet, dass** das Gerät (10) einen Bildschirm (14) zur Sichtbarmachung verwendet, der ein verarbeitetes Bild (13) liefert, in dem mindestens eine Kennung (11) eingeblendet erscheint, die das dominante Auge (12) der Person (2) anzeigt.

10. Verfahren nach Anspruch 9, **dadurch gekennzeichnet, dass** die Kennung ein Strich (11) ist, der das dominante Auge (12) der Person (2) mit dem optischen Fenster (18) der Zielvorrichtung (7) verbindet.

11. Verfahren nach einem der Ansprüche 1 bis 10, **dadurch gekennzeichnet, dass** die Zielvorrichtung (7) mit mindestens einer Peilmarkierung (20, 21) versehen ist, von denen mindestens eine (21) es ermöglicht, die Position des optischen Fensters (18) zu erkennen.

**12.** Verfahren nach einem der Ansprüche 1 bis 11, **dadurch gekennzeichnet, dass** das optische Fenster eine Durchgangsöffnung (18) ist.

**13.** Verfahren nach einem der Ansprüche 1 bis 12, **dadurch gekennzeichnet, dass** das Gerät eine ortsfeste Säule (10) ist, und dass das Ziel (6) auf der Säule (10) angeordnet ist.

**14.** Tragbare Zielvorrichtung (7) zur Durchführung eines Verfahrens nach einem der Ansprüche 1 bis 13, **dadurch gekennzeichnet, dass** sie eine Tafel (17) geringer Dicke enthält, die ein optisches Fenster (18) aufweist, wobei die Abmessungen der Tafel (17) mit einer bequemen Handhabung kompatibel sind, und dass die Tafel (17) einen Sockel (19) enthält, der mindestens eine Markierung (21) besitzt, die für die Position des optischen Fensters (18) repräsentativ ist, um es zu ermöglichen, das Fenster (18) in einem Bild (13) in dem Fall anzupeilen, in dem die Tafel (17) geneigt ist.

**15.** Vorrichtung nach Anspruch 14, **dadurch gekennzeichnet, dass** das optische Fenster (18) so bemessen ist, dass eine Person (2) das Ziel (6) nicht mit den beiden Augen gleichzeitig sehen kann, wobei die Tafel (17) den optischen Kegel teilweise bedeckt, dessen Basis von den zwei Augen der Person (2) begrenzt ist und dessen Spitze dem Ziel (6) entspricht.

**Claims**

**1.** Method for determining the dominant eye (12) of an individual (2), comprising the following steps:

- positioning the individual (2) in front of an apparatus (10) designed to acquire at least one frontal image (13) of said individual (2), then to process said image (13) and lastly to reconstruct information allowing the individual (2) to ascertain their dominant eye (12);
- viewing, by means of a sighting device (7) equipped with an optical window (18), a target (6) that is identifiable and discernible relative to the apparatus (10), this viewing being carried out through said window (18) and with both eyes open, the dimensions of said window (18) being such that they do not allow the individual (2) to see said target (6) with both eyes at the same time;
- acquiring, using the apparatus (10), at least one image (13) allowing at least the position of the two eyes of the individual (2) and of the optical window (18) to be seen;
- calculationally processing said at least one image (13), the calculations taking into account the position of a central point located between the two eyes of the individual (2), the position of the target (6) and the position of the window (18); and
- reconstructing, using the apparatus (10), the information indicating the dominant eye (12) of the individual (2).

**2.** Method according to Claim 1, **characterized in that** the sighting device (7) is portable, and **in that** the individual (2) manually adjusts the position of this device (7) in order to see the target (6).

**3.** Method according to either one of Claims 1 and 2, **characterized in that** the means for acquiring the image is a camcorder (3).

**4.** Method according to any one of Claims 1 to 3, **characterized in that** both eyes of the individual (2) and the optical window (18) appear simultaneously in a given image (13).

**5.** Method according to any one of Claims 1 to 3, **characterized in that** the apparatus (10) acquires at least two images (13), a first image that is a zoomed-out view in which both the eyes of the individual (2) and the optical window (18) feature at the same time, and a second image that is a zoomed-in view of the eyes of the individual (2) and that allows the position of a central point located between the eyes to be precisely identified, the processing of the first image being carried out on the basis of information gathered from the second image.

**6.** Method according to any one of Claims 1 to 5, **characterized in that** the individual (2) wears a pair of spectacles (22), to which a clip (24) equipped with at least one marker (23, 26) is fastened, at least one of the markers (23, 26) being intended to pinpoint a central point between the eyes.

**7.** Method according to any one of Claims 1 to 6, **characterized in that** the calculational processing is based on the position of a first straight line joining the target (6) and the window (18), and a second straight line connecting said target (6) to a central point located between the two eyes, said positions being determined relative to a reference straight line passing through the target (6) and lying parallel to the sighting axis of the means (3) for acquiring the image.

**8.** Method according to Claim 7, **characterized in that** the calculational processing comprises at least two steps, the first of which consists in determining a first angle ($\theta v$) between the first straight line and the reference straight line, and a second angle ($\theta c$) between the second straight line and said reference straight line, and the second step in which consists in comparing said angles to each other.

9. Method according to any one of Claims 1 to 8, **characterized in that** the apparatus (10) employs a display screen (14) to deliver a processed image (13) on which appears, overlaid, at least one identifier (11) indicating the dominant eye (12) of the individual (2).

10. Method according to Claim 9, **characterized in that** the identifier is a line (11) joining the dominant eye (12) of the individual (2) to the optical window (18) of the sighting device (7).

11. Method according to any one of Claims 1 to 10, **characterized in that** the sighting device (7) is equipped with at least one pinpointing marker (20, 21), at least one (21) of which allows the position of the optical window (18) to be identified.

12. Method according to any one of Claims 1 to 11, **characterized in that** the optical window is a through-orifice (18).

13. Method according to any one of Claims 1 to 12, **characterized in that** the apparatus is a fixed column (10), and **in that** the target (6) is placed on said column (10).

14. Portable sighting device (7) for implementing a method according to any one of Claims 1 to 13, **characterized in that** it comprises a thin board (17) comprising an optical window (18), the dimensions of said board (17) being compatible with easy manual manipulation, and **in that** the board (17) comprises a mounting (19) possessing at least one marker (21) representing the position of the optical window (18), in order to allow said window (18) to be pinpointed on an image (13) in the case where the board (17) is inclined.

15. Device according to Claim 14, **characterized in that** the optical window (18) is dimensioned so that an individual (2) cannot see the target (6) with both eyes at the same time, the board (17) partially covering their view cone, the base of which is bounded by the two eyes of the individual (2) and the tip of which corresponds to the target (6).

**Fig. 1**

**Fig. 3a**

**Fig. 3b**

**Fig. 2a**

**Fig. 2b**

**Fig. 4**

**EP 2 683 286 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5689619 A
- US 20090262302 A